(19) Europäisches Patentamt
European Patent Office
Office européen des brevets

(11) EP 3 246 029 A1

(12) **EUROPEAN PATENT APPLICATION**

(43) Date of publication:
**22.11.2017 Bulletin 2017/47**

(51) Int Cl.:
***A61K 31/496*** *(2006.01)* ***A61P 35/00*** *(2006.01)*
***A61P 35/04*** *(2006.01)* ***A61K 31/513*** *(2006.01)*

(21) Application number: **16170498.6**

(22) Date of filing: **19.05.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **BOEHRINGER INGELHEIM INTERNATIONAL GMBH**
**55216 Ingelheim am Rhein (DE)**

(72) Inventor: **HILBERG, Frank**
**1050 Vienna (AT)**

(74) Representative: **Simon, Elke Anna Maria et al**
**Boehringer Ingelheim GmbH**
**Binger Strasse 173**
**55216 Ingelheim am Rhein (DE)**

# (54) PHARMACEUTICAL COMBINATION OF NINTEDANIB AND CAPECITABINE FOR THE TREATMENT OF COLORECTAL CANCER

(57) The compounds

- 3-Z-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, and
- 5'-deoxy-5-fluoro-N-[(pentyloxy)carbonyl]-cytidine or a pharmaceutically acceptable salt thereof,

for combined use in the treatment of colorectal cancer.

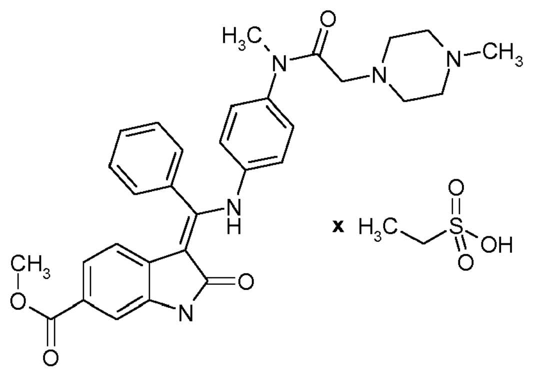

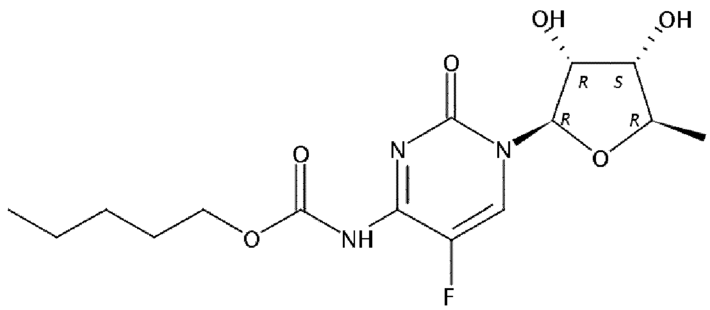

EP 3 246 029 A1

## Description

The present invention relates to a pharmaceutical combination useful for the treatment of diseases which involve cell proliferation, more specifically colorectal cancer. The invention also relates to a method for the treatment of diseases with high unmet medical need, comprising simultaneous, separate or sequential administration of effective amounts of specific active compounds and/or co-treatment with radiation therapy, in a ratio which provides an additive and/or synergistic effect, and to the combined use of these specific compounds and/or radiotherapy for the manufacture of corresponding pharmaceutical combination preparations.

The present invention relates more specifically to a pharmaceutical combination comprising the compound nintedanib (Compound A) or a pharmaceutically acceptable salt thereof and Capecitabine (Compound B) or a pharmaceutically acceptable salt thereof, optionally in combination with radiotherapy.

## Background to the invention

In the treatment of colorectal cancer various combination treatments have been assessed so far including the combination of capecitabine and becacizumab (*see,* e.g. Tebutt et al., J.Clin.Oncol. (2010), 28:3191-3198). The combination improved progression free survival but did not improve response rate or overall survival. A further downside is the fact that bevacizumab requires frequent hospital visits.

Nintedanib, the compound 3-Z-[1-(4-(*N*-((4-methyl-piperazin-1-yl)-methylcarbonyl)-*N*-methyl-amino)-anilino)-1-phenyl-methylene]-6-methoxycarbonyl-2-indolinone (Compound A) is an innovative compound having valuable pharmacological properties, especially for the treatment of oncological diseases, immunologic diseases or pathological conditions involving an immunologic component, or fibrotic diseases.

The chemical structure of this compound is depicted below as Formula A.

**Formula A**

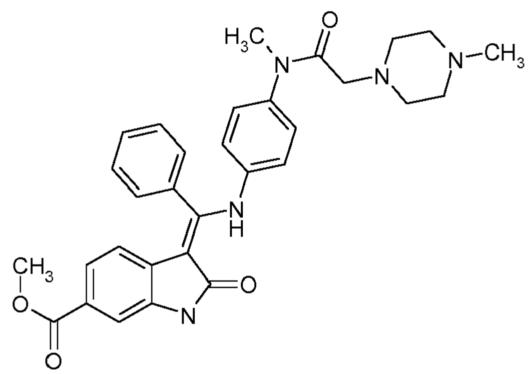

The base form of this compound is described in WO 01/27081, the monoethanesulphonate salt form is described in WO 2004/013099 and various further salt forms are presented in WO 2007/141283. The use of this molecule for the treatment of immunologic diseases or pathological conditions involving an immunologic component is being described in WO 2004/017948, the use for the treatment of oncological diseases is being described in WO 2004/096224 and the use for the treatment of fibrotic diseases is being described in WO 2006/067165.

The monoethanesulphonate salt form of this compound presents properties which makes this salt form especially suitable for development as medicament. The chemical structure of 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone-monoethanesulphonate is depicted below as Formula A1.

**Formula A1**

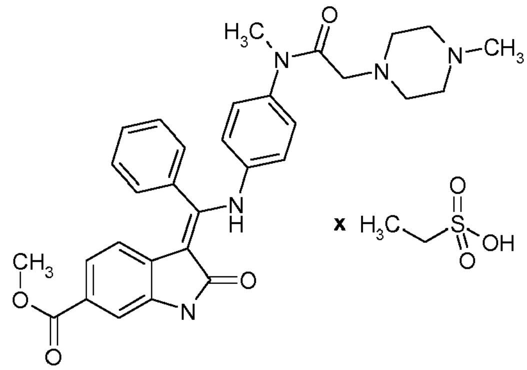

Preclinical studies have shown that this compound is a highly potent, orally bioavailable inhibitor of vascular endothelial growth factor receptors (VEGFRs), platelet-derived growth factor receptors (PDGFRs) and fibroblast growth factor receptors (FGFRs) that suppresses tumor growth through mechanisms inhibiting tumor neovascularization and tumor stromal components. It has further been shown that this compound inhibits signaling in endothelial- and smooth muscle cells and pericytes, and reduces tumor vessel density.

Furthermore, this compound shows *in vivo* anti-tumor efficacy in all models tested so far at well tolerated doses. The following table shows the results of the *in vivo* anti-tumor efficacy testing in xenograft models and in a syngeneic rat tumor model.

| Cancer | Model | Efficacy |
|---|---|---|
| Colorectal | HT-29<br>HT-29 large tumors | T/C 16% @ 100mg/kg/d<br>tumor volume reduction |
| Glioblastoma | GS-9L syngeneic rat | T/C 32% @ 50mg/kg/d |
| Head and neck | FaDu | T/C 11% @ 100mg/kg/d |
| Lung (non-small-cell) | NCI-H460<br>Calu-6 | T/C 54% @ 25mg/kg/d<br>T/C 24% @ 50mg/kg/d |
| Ovarian | SKOV3 | T/C 19% @ 50mg/kg/d |
| Prostate (hormone-dependent) | PAC-120 | T/C 34% @ 100mg/kg/d |
| Renal | Caki-1 | T/C 13% @ 100mg/kg/d |
| Pancreas (murine transgenic) | Rip-Tag | interference with tumor formation |
| T/C represents the reduction of tumor size in % of the control | | |

This compound is thus suitable for the treatment of diseases in which angiogenesis or the proliferation of cells is involved. It has been approved for idiopatic pulmonary fibrosis, e.g. in the US, Japan and for the EU. In Europe it has been also approved for Non-Small-Cell Lung Cancer/Carcinoma (NSCLC). A global phase III study, called as the LUME-Colon 1 study , comparing Nintedanib+BSC monotherapy versus placebo+BSC in pts with mCRC refractory to standard therapies is ongoing (NCT02149108).

Compound B

Compound B is capecitabine according to Formula B

**Formula B**

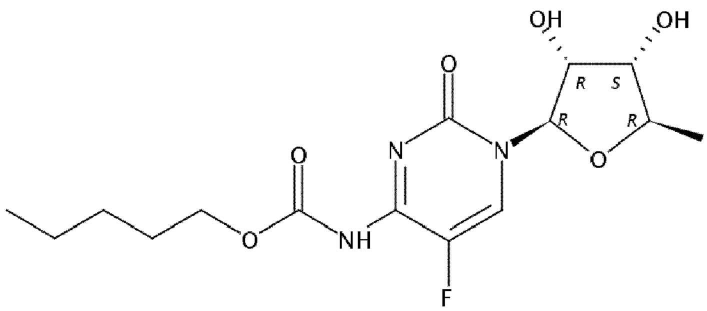

Capecitabine, or 5'-deoxy-5-fluoro-N-[(pentyloxy)carbonyl]-cytidine, has the CAS Registry Number 154361-50-9, is marketed as Xeloda®.
Capecitabine is an oral medication for treating advanced breast cancer that is resistant to combination therapy with the drugs of choice, paclitaxel (Taxol®) and a drug from the anthracycline family of drugs, for example, doxorubicin (Adriamycin®). Enzymes convert capecitabine to 5-fluorouracil (FU or 5-FU) in vivo. Both normal and tumor cells metabolize 5-FU to 5-fluoro-2'-deoxyuridine monophosphate (FdUMP) and 5-fluorouridine triphosphate (FUTP). These metabolites cause cell injury by two different mechanisms. First, FdUMP and the folate cofactor, NS-10-methylenetetrahydrofolate, bind to thymidylate synthase (TS) to form a covalently bound ternary complex. This binding inhibits the formation of thymidylate from 2'-deoxyuridylate. Thymidylate is the necessary precursor of thymidine triphosphate, which is essential for the synthesis of DNA, so that a deficiency of this compound can inhibit cell division. Second, nuclear transcriptional enzymes can mistakenly incorporate FUTP in place of uridine triphosphate (UTP) during the synthesis of RNA. This metabolic error can interfere with RNA processing and protein synthesis.
Capecitabine was approved by the FDA in 1998 for the treatment of breast cancer and in 2005 for the treatment of colorectal cancer. Capecitabine is approved in the United States for the treatment of metastatic colorectal and breast cancer. Adverse effects reported with capecitabine include diarrhea, vomiting, nausea, mouth sores, hand-foot syndrome, fever, and infection, according to the FDA.
Capecitabine is an oral prodrug that is converted to its only active metabolite, FU (5-fluoro-uracil), by thymidine phosphorylase. Higher levels of this enzyme are found in several tumors and the liver, compared with normal healthy tissue. In adults, capecitabine has a bioavailability of ≈100% with a Cmax of 3.9 mg/L, Tmax of 1.5 to 2 hr, and AUC of 5.96 mg·h/L. The predominant route of elimination is renal, and dosage reduction of 75% is recommended in patients with creatinine clearance (CrCl) of 30 to 50 mL/min. The drug is contraindicated if CrCl is <30 mL/min. Capecitabine has shown varying degrees of efficacy with acceptable tolerability in numerous cancers including prostate, renal cell, ovarian, and pancreatic, with the largest amount of evidence in metastatic breast and colorectal cancer. The dose of capecitabine approved by the US Food and Drug Administration (FDA) for both metastatic colorectal and breast cancer is 1250 mg/$m^2$ given orally twice per day (bid), usually separated by 12 hours for the first 2 weeks of every 3-week cycle. (A review on capacetabine; World journal of pharmaceutical research; 4 (7):1427-1432)

**Summary of the invention**

One embodiment of the invention is a pharmaceutical combination comprising components

(i) 3-Z-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, and
(ii) 5'-deoxy-5-fluoro-N-[(pentyloxy)carbonyl]-cytidine or a pharmaceutically acceptable salt thereof.

A further preferred embodiment of the invention is the pharmaceutical combination of the preceding paragraph, in which the pharmaceutically acceptable salt of the compound 3-Z-[1-(4-(*N*-((4-methyl-piperazin-1-yl)-methylcarbonyl)-*N*-methyl-amino)-anilino)-1-phenyl-methylene]-6-methoxycarbonyl-2-indolinone is its monoethanesulphonate salt form.
Preferred is the pharmaceutical combination according to one of the preceding paragraphs, wherein components (i) and (ii) is for simultaneous, separate, or sequential use, even more preferred for separate or sequential use.
A further preferred embodiment of the invention is the pharmaceutical combination of one of the preceding paragraphs which is further adapted for a co-treatment with radiotherapy.

Preferred is the pharmaceutical combination according to one of the preceding paragraphs for use in the treatment of colorectal cancer, more preferred metastatic colorectal cancer, even more preferred metastatic colorectal cancer refractory or intolerant to standard therapies. The standard therapies are preferably selected from the group consisting of fluoropyrimidine, irinotecan, oxaliplatin, anti-angiogenesis inhibitor and anti-EGFR antibody (if wild-type RAS).

A further preferred embodiment of the invention is the pharmaceutical combination of one of the preceding paragraphs, wherein component (i) is intended to be administered in the amount of 100 mg of the free base, twice daily, more preferred 150 mg of the free base, twice daily, most preferred 200 mg of the free base, twice daily.

Most preferred is an embodiment of the invention is the pharmaceutical combination of one of the preceding paragraphs, wherein component (ii) is administered in the amount of 1000 mg per $m^2$, twice daily, on day 1-14 every 21 days. If the treatment regimen requires that both compound A and B are to be administered at a certain day, a fixed-dose combination of compound A and B in one formulation or dosage form can be administered to the patient (fixed-dose combination). In another embodiment of the invention compound A and B are not contained together in a single formulation or dosage form and intended to be administered separately, more preferred sequentially, i.e. one after the other.

A typical treatment regimen could look as follows:

- Day 1-14, 200 mg compound A calculated as free base, twice daily and 1000 mg per $m^2$ compound B, twice daily.
- Day 15- 21, 200 mg compound A calculated as free base, twice daily.
- Day 22-35, repeat Day 1-14 treatment.
- Day 36-42, repeat Day 15-21 treatment.

The above treatment cycle may be repeated or modified as needed depending on the patient's response to the drug.

A further embodiment of the invention are compounds

(iii) 3-Z-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, and

(iv) 5'-deoxy-5-fluoro-N-[(pentyloxy)carbonyl]-cytidine or a pharmaceutically acceptable salt thereof, and

for combined use in the treatment of colorectal cancer.

A further embodiment of the invention is a method for treating metastatic colorectal cancer comprising administering to a patient in need thereof compound (iii): 3-*Z*-[[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, in combination with compound (iv): 5'-deoxy-5-fluoro-N-[(pentyloxy)carbonyl]-cytidine or a pharmaceutically acceptable salt thereof.

A further embodiment of the invention is a method for treating metastatic colorectal cancer comprising administering to a patient in need thereof

a pharmaceutical combination consisting essentially of compound (iii), compound (iv).

A further embodiment of the invention is a method for treating metastatic colorectal cancer comprising administering to a patient in need thereof

a pharmaceutical combination consisting of compound (iii), compound (iv).

A further embodiment of the invention is a method for treating metastatic colorectal cancer comprising

- identifying a patient in need of treatment of colorectal cancer
- treating the patient with a combination therapy comprising compound (iii), and compound (iv).

**Detailed description of the invention**

As already mentioned hereinbefore, the present invention relates to a pharmaceutical combination comprising an effective amount of the Compound A or a pharmaceutically acceptable salt thereof and an effective amount of the Compound mixture B or a pharmaceutically acceptable salt thereof.

A combination treatment of the present invention as defined herein may be achieved by way of the simultaneous, sequential or separate administration of the individual components of said treatment. A combination treatment as defined herein may be applied as a sole therapy or may involve surgery or radiotherapy or an additional chemotherapeutic or targeted agent in addition to a combination treatment of the invention. Surgery may comprise the step of partial or complete tumor resection, prior to, during or after the administration of the combination treatment as described herein.

According to one aspect of the invention the treatment does not require many hospital visits as would be the case if one or more components of the combination were to be administered by injection, more particularly intravenous

injection. In fact, the combination is for use in an oral administration regime.

According to another aspect of the present invention, the effect of a method of treatment of the present invention is expected to be at least equivalent to the addition of the effects of each of the components of said treatment used alone, that is, of each of the compounds and ionising radiation used alone. The effect can be show by the TGI value in a corresponding xenograft mouse model or in the response rate (RR), disease control (DC), progression free survival (PFS) or overall survival (OS), the extent of the response, the duration of response, the stabilization rate, the duration of stabilization in a clinical trial.

According to another aspect of the present invention the effect of a method of treatment of the present invention is expected to be greater than the addition of the effects of each of the components of said treatment used alone, that is, of each of the compounds and ionising radiation used alone.

According to another aspect of the present invention the effect of a method of treatment of the present invention is expected to be a synergistic effect. A combination treatment is defined as affording a synergistic effect if the effect is therapeutically superior, as measured by, for example, the extent of the response, the duration of response, the response rate, the stabilization rate, the duration of stabilization, the time to disease progression, the progression free survival or the overall survival, to that achievable on dosing one or other of the components of the combination treatment at its conventional dose. For example, the effect of the combination treatment is synergistic if the effect is therapeutically superior to the effect achievable with one component alone. Further, the effect of the combination treatment is synergistic if a beneficial effect is obtained in a group of patients that does not respond (or responds poorly) to one component alone. In addition, the effect of the combination treatment is defined as affording a synergistic effect if one of the components is dosed at its conventional dose and the other component(s) is/are dosed at a reduced dose and the therapeutic effect, as measured by, for example, the extent of the response, the duration of response, the response rate, the stabilization rate, the duration of stabilization, the time to disease progression, the progression free survival or the overall survival, is equivalent to that achievable on dosing conventional amounts of the components of the combination treatment.

In particular, synergy is deemed to be present if the conventional dose of one of the components may be reduced without detriment to one or more of the extent of the response, the duration of response, the response rate, the stabilization rate, the duration of stabilization, the time to disease progression, the progression free survival or the overall survival,, in particular without detriment to the duration of the response, but with fewer and/or less troublesome side-effects than those that occur when conventional doses of each component are used.

The advantages of the present invention are the potential for an improved clinical benefit for cancer patients treated with this pharmaceutical combination involving one or more of the following mechanisms:

Additive or synergistic antitumor effect mediated by the combination of two different anticancer principles and target structures: Compound A1 is an antiangiogenic compound targeting the tumor vasculature (endothelial cells, pericytes, and smooth muscle cells) with suppression of tumor (re-)growth and metastatic spread; Compound B is a 5-FU prodrug. Unlike normal cells, cancer cells are genetically instable, causing them to replicate inaccurately. As tumors progress, this genetic instability leads to subpopulations of tumor cells with different biological features. An antitumor treatment like Compound B may terminate even the majority of tumor tissue. However, finally, some cell clones will become refractory. After the treatment-sensitive cells have been killed, the resistant cells may rapidly divide again to restore a tumor that is inherently resistant to the therapy. Therefore, simultaneous targeting of different principles driving cancer growth and spread with the described combination of Compound A1 and Compound B reduces the risk of primary and secondary tumor resistance and tumor escape as well. The validity of such approaches has been demonstrated for combination and multimodality treatment in a variety of solid and hematologic human malignancies, but not for the combination object of the present invention, i.e. the combination of Compound A1 and Compound B. Of importance in the context of the present invention may be the fact that Compound A1 primarily acts on the genetically stable cells of the tumor vasculature which are less prone to spontaneous mutation and resistance development as compared to the malignant cells.

**Example A:**

*In vivo* tumor xenograft experiment design to explore the interaction potential between nintedanib and capecitabine

In order to analyse the anti-tumor effects of combining the inhibition of tumor angiogenesis by interfering with the VEGFR, FGFR and PDGFR signalling cascades with the established anti-proliferative treatment modality of capecitabine (Xeloda®) in a xenograft model of CRC the following in vivo experiment is performed. Nude mice carrying established subcutaneous HT-29 xenografts (human CRC tumor cell line) are randomized and treated with either nintedanib or capecitabine alone or with the combination of both drugs. After 28 days of treatment the tumors on the control treated mice reach the endpoint and are in average -1200 $mm^3$ in volume. The results show that the combination of suboptimal doses of nintedanib and capecitabine results in improved antitumour efficacy with a TGI value of 95% compared to single

agent treatments (TGI values of 35% and 50 %, respectively).

$$TGI = \left(1 - \frac{Xtreated, final - Xtreated, day\ 1}{Xcontrol, final - Xcontrol,\ \ day\ 1}\right) * 100\%$$

where X = mean tumor weigh, final is day 28

The results show that the doses applied during this tumor experiment does not lead to weight loss in the treated mice. The weight gain of the mice in the treatment groups in comparison to the weight of the control mice is reduced, but nevertheless well tolerated. The combined use of nitedanib and capetcitbine significantly improves the antitumor efficacy on HT-29 xenograft mice compared to either nintedanib or capecitabine alone.

**Example B:**

An open-label randomized phase II study to assess the efficacy and safety of nintedanib alone or in combination with capecitabine for patients with refractory metastatic colorectal cancer

This trial (1199.251) investigates the efficacy and safety of nintedanib alone or in combination with capecitabine for patients with refractory mCRC after failure of at least two lines of standard treatment. The outcome of this study should be informative for future trials to investigate this dual oral combination therapy.

The primary objective is to assess progression free survival (PFS) in patients treated with nintedanib alone or in combination with capecitabine, Secondary objectives: assessment of overall survival (OS), objective response rate (ORR), disease control (DC), and safety in both treatment arms. ORR means objective response rate, OR is CR+PR , i.e. those patients with complete response and partial response. DC means disease control, DC = CR+PR+SD, i.e. those patience havin a complete response, partial response or stable diseasae.

Open label randomized Phase II trial, 1:1 randomization, primary endpoint will be PFS based on investigator assessments according to Response Evaluation Criteria In Solid Tumors (RECIST) 1.1. Randomization will be stratified by time from diagnosis of metastatic disease until randomization in the trial (<24 months vs 24 months or more).

Main criteria for inclusion:

- Diagnosis of mCRC
- Prior therapy with a fluoropyrimidines-, oxaliplatin- and irinotecan-based chemotherapy, an anti-VEGF biological therapy, and if RAS wild-type, an anti-EGFR therapy.
- Prior use of regorafenib and/or TAS-102 is allowed.
- ECOG performance status 0-1
- Adequate organ function
- No prior nintedanib treatment

Treatment is projected until disease progression according to RECIST 1.1, unacceptable toxicity, or patient refusal.

Primary endpoint: PFS

Secondary endpoints: OS, OR, DC, and the percentage of patients with grade 3 or worse adverse events

Safety criteria: Frequency and severity of adverse events according to Common Terminology Criteria for Adverse Events (CTCAE) Version 4.03; Changes in safety laboratory parameters.

Statistical methods: PFS and OS will be analyzed using the Peto method for calculating the hazard ratio (HR) adjusted for the stratification factor used at randomization and its 95% confidence interval (CI). Objective Response rates and disease control rate with their corresponding 95% Wilson confidence intervals will be presented. The percentage of patients with CTCAE grade 3 or worse AEs will be presented along with their corresponding asymptotic 2-sided 95% CIs. Other endpoints will be assessed using descriptive statistics.

**Further embodiments**

Further pharmaceutically acceptable salts of the compounds of the combination in accordance with the present invention than those already described hereinbefore may, for example, include acid addition salts. Such acid addition

salts include, for example, salts with inorganic or organic acids affording pharmaceutically acceptable anions such as with hydrogen halides or with sulphuric or phosphoric acid, or with trifluoroacetic, citric or maleic acid. In addition, pharmaceutically acceptable salts may be formed with an inorganic or organic base which affords a pharmaceutically acceptable cation. Such salts with inorganic or organic bases include for example an alkali metal salt, such as a sodium or potassium salt and an alkaline earth metal salt such as a calcium or magnesium salt.

In accordance with the present invention, the compounds of the combination may be formulated using one or more pharmaceutically acceptable excipients or carriers, as suitable. Suitable formulations for both compounds A1 and B1 which may be used within the scope of the present invention have already been described in the literature and in patent applications related to these compounds. These formulations are incorporated herein by reference.

In a further preferred embodiment in accordance with the present invention, the formulation for the compound of formula A1 is a lipid suspension of the active substance comprising preferably a lipid carrier, a thickener and a glidant/solubilizing agent, most preferably in which the lipid carrier is selected from corn oil glycerides, diethylenglycolmonoethylether, ethanol, glycerol, glycofurol, macrogolglycerolcaprylocaprate, macrogolglycerollinoleate, medium chain partial glycerides, medium chain triglycerides, polyethylene glycol 300, polyethylene glycol 400, polyethylene glycol 600, polyoxyl castor oil, polyoxyl hydrogenated castor oil, propylene glycol monocaprylate, propylene glycol monolaurate, refined soybean oil, triacetin, triethyl citrate, or mixtures thereof, the thickener is selected from oleogel forming excipients, such as Colloidal Silica or Bentonit, or lipophilic or amphiphilic excipients of high viscosity, such as polyoxyl hydrogenated castor oil, hydrogenated vegetable oil macrogolglycerol-hydroxystearates, macrogolglycerol-ricinoleate or hard fats, and the glidant/solubilizing agent is selected from lecithin, optionally further comprising one or more macrogolglycerols, preferably selected from macrogolglycerol-hydroxystearate or macrogolglycerol-ricinoleate. The lipid suspension formulation may be prepared by conventional methods of producing formulations known from the literature, i.e. by mixing the ingredients at a pre-determined temperature in a pre-determined order in order to obtain a homogenized suspension.

The above formulation may be preferably incorporated in a pharmaceutical capsule, preferably a soft gelatin capsule, characterised in that the capsule shell comprises e.g. glycerol as plasticizing agent, or a hard gelatin or hydroxypropylmethylcellulose (HPMC) capsule, optionally with a sealing or banding. The capsule pharmaceutical dosage form may be prepared by conventional methods of producing capsules known from the literature. The soft gelatin capsule may be prepared by conventional methods of producing soft gelatin capsules known from the literature, such as for example the "rotary die procedure", described for example in Swarbrick, Boylann, Encyclopedia of pharmaceutical technology, Marcel Dekker, 1990, Vol. 2, pp 269 ff or in Lachmann et al., "The Theory and Practice of Industrial Pharmacy", 2nd Edition, pages 404-419, 1976, or other procedures, such as those described for example in Jimerson R. F. et al., "Soft gelatin capsule update", Drug Dev. Ind. Pharm., Vol. 12, No. 8-9, pp. 1133-44, 1986.

The above defined formulation or the above defined capsule may be used in a dosage range of from 0.1 mg to 20 mg of active substance/ kg body weight, preferably 0.5 mg to 4 mg active substance /kg body weight. The amount of active substance is calculated as free base.

The above defined capsules may be packaged in a suitable glass container or flexible plastic container, or in an aluminium pouch or double poly bag.

The following examples of carrier systems (formulations), soft gelatin capsules, bulk packaging materials, and of a manufacturing process are illustrative of the present invention and shall in no way be construed as a limitation of its scope.

**Examples of carrier systems (formulations), soft gelatin capsules, bulk packaging materials, and of a manufacturing process for the preparation of a lipid suspension formulation of compound A1**

The active substance in all the Examples 1 to 10 is 3-Z-[1-(4-(N-((4-methyl-piperazin-1-yl)-methylcarbonyl)-N-methyl-amino)-anilino)-1-phenyl-methylene]-6-methoxycarbonyl-2-indolinone-monoethanesulphonate (compound A1).

Example 1

Lipid based carrier system

| **Formulation** | **A** | **B** | **C** |
|---|---|---|---|
| **Ingredients** | **[%]** | **[%]** | **[%]** |
| Active Substance | 43.48 | 43.48 | 43.48 |
| Triglycerides, Medium-Chain | 28.70 | 37.83 | 38.045 |

(continued)

| Formulation | A | B | C |
|---|---|---|---|
| Ingredients | [%] | [%] | [%] |
| Hard fat | 27.39 | 18.26 | 18.26 |
| Lecithin | 0.43 | 0.43 | 0.215 |
| Total (Fillmix) | 100.00 | 100.00 | 100.00 |

**Example 2**

Lipid based carrier system with additional surfactant

| Ingredients | [%] |
|---|---|
| Active Substance | 42.19 |
| Triglycerides, Medium-Chain | 41.77 |
| Hard fat | 12.66 |
| Cremophor RH40 | 2.95 |
| Lecithin | 0.42 |
| Total (Fillmix) | 100.00 |

**Example 3**

Hydrophilic carrier system

| Ingredients | [%] |
|---|---|
| Active Substance | 31.75 |
| Glycerol 85% | 3.17 |
| Purified Water | 4.76 |
| Macrogol 600 | 58.10 |
| Macrogol 4000 | 2.22 |
| Total (Fillmix) | 100.00 |

**Example 4**

Soft gelatin capsule containing 50 mg of active substance (free base)

| | | Formulation A | Formulation B | Formulation C |
|---|---|---|---|---|
| Ingredients | Function | mg per capsule | mg per capsule | mg per capsule |
| Active Substance | Active Ingredient | 60.20 | 60.20 | 60.20 |
| Triglycerides, Medium-chain | Carrier | 40.95 | 53.70 | 54.00 |
| Hard fat | Thickener | 38.25 | 25.50 | 25.50 |
| Lecithin | Wetting agent / Glidant | 0.60 | 0.60 | 0.30 |

(continued)

| | | Formulation A | Formulation B | Formulation C |
|---|---|---|---|---|
| **Ingredients** | **Function** | **mg per capsule** | **mg per capsule** | **mg per capsule** |
| Gelatin | Film-former | 72.25 | 72.25 | 72.25 |
| Glycerol 85% | Plasticizer | 32.24 | 32.24 | 32.24 |
| Titanium dioxide | Colorant | 0.20 | 0.20 | 0.20 |
| Iron oxide A | Colorant | 0.32 | 0.32 | 0.32 |
| Iron oxide B | Colorant | 0.32 | 0.32 | 0.32 |
| **Total Capsule Weight** | | **245.33** | **245.33** | 245.33 |

**Example 5**

Soft gelatin capsule containing 100 mg of active substance

| | | Formulation A | Formulation B | Formulation C |
|---|---|---|---|---|
| **Ingredients** | **Function** | **mg per capsule** | **mg per capsule** | **mg per capsule** |
| Active Substance | Active Ingredient | 120.40 | 120.40 | 120.40 |
| Triglycerides, Medium-chain | Carrier | 81.90 | 107.40 | 106.8 |
| Hard fat | Thickener | 76.50 | 51.00 | 51.00 |
| Lecithin | Wetting agent / Glidant | 1.20 | 1.20 | 1.80 |
| Gelatin | Film-former | 111.58 | 111.58 | 111.58 |
| Glycerol 85% | Plasticizer | 48.79 | 48.79 | 48.79 |
| Titanium dioxide | Colorant | 0.36 | 0.36 | 0.36 |
| Iron oxide A | Colorant | 0.06 | 0.06 | 0.06 |
| Iron oxide B | Colorant | 0.17 | 0.17 | 0.17 |
| **Total Capsule Weight** | | **440.96** | **440.96** | **440.96** |

**Example 6**

Soft gelatin capsule containing 125 mg of active substance

| | | Formulation A | Formulation B | Formulation C |
|---|---|---|---|---|
| **Ingredients** | **Function** | **mg per capsule** | **mg per capsule** | **mg per capsule** |
| Active Substance | Active Ingredient | 150.50 | 150.50 | 150.50 |
| Triglycerides, Medium-chain | Carrier | 102.375 | 134.25 | 133.5 |
| Hard fat | Thickener | 95.625 | 63.75 | 63.75 |
| Lecithin | Wetting agent / Glidant | 1.50 | 1.50 | 2.25 |
| Gelatin | Film-former | 142.82 | 142.82 | 142.82 |
| Glycerol 85% | Plasticizer | 62.45 | 62.45 | 62.45 |
| Titanium dioxide | Colorant | 0.47 | 0.47 | 0.47 |
| Iron oxide A | Colorant | 0.08 | 0.08 | 0.08 |

(continued)

| | | Formulation A | Formulation B | Formulation C |
|---|---|---|---|---|
| **Ingredients** | **Function** | **mg per capsule** | **mg per capsule** | **mg per capsule** |
| Iron oxide B | Colorant | 0.22 | 0.22 | 0.22 |
| **Total Capsule Weight** | | **556.04** | **556.04** | **556.04** |

**Example 7**

Soft gelatin capsule containing 150 mg of active substance

| | | Formulation A | Formulation B | Formulation C |
|---|---|---|---|---|
| **Ingredients** | **Function** | **mg per capsule** | **mg per capsule** | **mg per capsule** |
| Active Substance | Active Ingredient | 180.60 | 180.60 | 180.60 |
| Triglycerides, Medium-chain | Carrier | 122.85 | 161.10 | 160.20 |
| Hard fat | Thickener | 114.75 | 76.50 | 76.50 |
| Lecithin | Wetting agent / Glidant | 1.80 | 1.80 | 2.70 |
| Gelatin | Film-former | 142.82 | 142.82 | 142.82 |
| Glycerol 85% | Plasticizer | 62.45 | 62.45 | 62.45 |
| Titanium dioxide | Colorant | 0.47 | 0.47 | 0.47 |
| Iron oxide A | Colorant | 0.08 | 0.08 | 0.08 |
| Iron oxide B | Colorant | 0.22 | 0.22 | 0.22 |
| **Total Capsule Weight** | | **626.04** | **626.04** | **626.04** |

**Example 8**

Soft gelatin capsule containing 200 mg of active substance

| | | Formulation A | Formulation B | Formulation C |
|---|---|---|---|---|
| **Ingredients** | **Function** | **mg per capsule** | **mg per capsule** | **mg per capsule** |
| Active Substance | Active Ingredient | 240.80 | 240.80 | 240.80 |
| Triglycerides, Medium-chain | Carrier | 163.30 | 214.80 | 216.00 |
| Hard fat | Thickener | 153.50 | 102.00 | 102.00 |
| Lecithin | Wetting agent / Glidant | 2.40 | 2.40 | 1.20 |
| Gelatin | Film-former | 203.19 | 203.19 | 203.19 |
| Glycerol 85% | Plasticizer | 102.61 | 102.61 | 102.61 |
| Titanium dioxide | Colorant | 0.57 | 0.57 | 0.57 |
| Iron oxide A | Colorant | 0.90 | 0.90 | 0.90 |
| Iron oxide B | Colorant | 0.90 | 0.90 | 0.90 |
| **Total Capsule Weight** | | **868.17** | **868.17** | **868.17** |

**Example 9**

Bulk packaging materials for the packaging of the soft gelatin capsules of above examples 1 to 4 may be aluminium pouches or double poly bags.

**Example 10**

In the following, a manufacturing process for the preparation of a lipid suspension formulation of the active substance and a process for the encapsulation are described.

aHard fat and parts of Medium-chain triglycerides are pre-mixed in the processing unit. Subsequently lecithin, the rest of medium-chain triglycerides and the active substance are added. The suspension is mixed, homogenized, deaerated and finally sieved to produce the formulation (Fillmix).

b The gelatin basic mass components are mixed and dissolved at elevated temperature. Then, the corresponding colours and additional water are added and mixed, producing the Coloured Gelatin Mass.

c After adjustment of the encapsulation machine, Fillmix and Coloured Gelatin Mass are processed into soft gelatin capsules using the rotary-die process. This process is e.g. described in Swarbrick, Boylann, Encyclopedia of pharmaceutical technology, Marcel Dekker, 1990, Vol. 2, pp 269 ff.

d After encapsulation, the traces of the lubricant medium-chain triglycerides are removed from the capsule surface, using ethanol denatured with acetone, containing small quantities of Phosal® 53 MCT, used here as anti-sticking agent.

e The initial drying is carried out using a rotary dryer. For the final drying step, capsules are placed on trays. Drying is performed at 15 - 26°C and low relative humidity.

f After 100% visual inspection of the capsules for separation of deformed or leaking capsules, the capsules are size sorted and further washed using ethanol denatured with acetone.

g Finally, the capsules are imprinted, using an Offset printing technology or an Ink-jet printing technology. Alternatively, the capsule imprint can be made using the Ribbon printing technology, a technology in which the gelatin bands are imprinted prior to the encapsulation step c.

Compound B (capecitabine, Xeloda®) may be administered according to known clinical practice.

The dosages and schedules may vary according to the particular disease state and the overall condition of the patient. Dosages and schedules may also vary if, in addition to a combination treatment of the present invention, one or more additional chemotherapeutic agents is/are used. Scheduling can be determined by the practitioner who is treating any particular patient.

Radiotherapy may be administered according to the known practices in clinical radiotherapy. The dosages of ionising radiation will be those known for use in clinical radiotherapy. The radiation therapy used will include for example the use of y-rays, X-rays, and/or the directed delivery of radiation from radioisotopes. Other forms of DNA damaging factors are also included in the present invention such as microwaves and UV- irradiation. For example X-rays may be dosed in daily doses of 1.8-2.0 Gy, 5 days a week for 5-6 weeks. Normally a total fractionated dose will lie in the range 45-60 Gy. Single larger doses, for example 5-10 Gy may be administered as part of a course of radiotherapy. Single doses may be administered intraoperatively. Hyperfractionated radiotherapy may be used whereby small doses of X-rays are administered regularly over a period of time, for example 0.1 Gy per hour over a number of days. Dosage ranges for radioisotopes vary widely, and depend on the half-life of the isotope, the strength and type of radiation emitted, and on the uptake by cells.

The size of the dose of each therapy which is required for the therapeutic or prophylactic treatment of a particular disease state will necessarily be varied depending on the host treated, the route of administration and the severity of the illness being treated. Accordingly the optimum dosage may be determined by the practitioner who is treating any particular patient. For example, it may be necessary or desirable to reduce the above-mentioned doses of the components of the combination treatments in order to reduce toxicity.

**Claims**

**1.** Pharmaceutical combination comprising components

(i) 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, and

(ii) 5'-deoxy-5-fluoro-N-[(pentyloxy)carbonyl]-cytidine or a pharmaceutically acceptable salt thereof.

**2.** Pharmaceutical combination according to claim 1, in which the pharmaceutically acceptable salt of the compound 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone is its monoethanesulphonate salt form.

**3.** Pharmaceutical combination according to claim 1 or 2, adapted for oral administration.

**4.** The pharmaceutical combination according to any one of claims 1 to 3, wherein component (i) and (ii) form a mixture for simultaneous use of both components.

**5.** The pharmaceutical combination according to any one of claims 1 to 4, which is further adapted for a co-treatment with radiotherapy.

**6.** The pharmaceutical combination according to any one of claims 1 to 5, for use in the treatment of colorectal cancer.

**7.** The pharmaceutical combination according to any one of claims 1 to 6, for use in the treatment of metastatic colorectal cancer.

**8.** The pharmaceutical combination according to any one of claims 1 to 7, for use in the treatment of metastatic colorectal cancer (CRC) patients, who have been previously treated with available therapies including fluoropyrimidine-, oxaliplatin- and irinotecan-based chemotherapies, anti-VEGF agents, and anti-EGFR agents.

**9.** The pharmaceutical combination according to any one of claims 1 to 8, wherein component (i) according to claim 1 is intended to be administered in an amount of 200 mg of the free base, twice daily.

**10.** The pharmaceutical combination according to any one of claims 1 to 8, wherein component (i) according to claim 1 is intended to be administered in an amount of 150 mg of the free base, twice daily.

**11.** The pharmaceutical combination according to any one of claims 1 to 8, wherein component (i) according to claim 1 is intended to be administered in an amount of 100 mg of the free base, twice daily.

**12.** The pharmaceutical combination according to any one of claims 1 to 11, wherein component (ii) according to claim 1 is intended to be administered in an amount of 1000 mg of the free base per $m^2$, twice daily at days 1 to 14 of a treatment cycle of 21 days.

**13.** The compounds

(iii) 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, and
(iv) 5'-deoxy-5-fluoro-N-[(pentyloxy)carbonyl]-cytidine or a pharmaceutically acceptable salt thereof,

for combined use in the treatment of colorectal cancer.

**14.** The compounds according to claim 13, in which the pharmaceutically acceptable salt of the compound 3-Z-[1-(4-(*N*-((4-methyl-piperazin-1-yl)-methylcarbonyl)-*N*-methyl-amino)-anilino)-1-phenyl-methylene]-6-methoxycarbonyl-2-indolinone is its monoethanesulphonate salt form.

**15.** The compounds according to claims 13 and 14, wherein compounds (iii) and (iv) are for simultaneous, separate or sequential use.

**16.** The compounds according to any one of claims claim 13 to 15, for use in a co-treatment with radiotherapy.

**17.** The compounds according to any one of claims 13 to 16, for use in the treatment of metastatic colorectal cancer.

**18.** The compounds according to any one of claims 13 to 17 for use in the treatment of metastatic colorectal cancer (CRC) patients, who have been previously treated with, or are not considered candidates for, available therapies including fluoropyrimidine-, oxaliplatin- and irinotecan-based chemotherapies, anti-VEGF agents, and anti-EGFR agents.

**19.** The compounds according to any one of claims 13 to 18, wherein compound (iii) according to claim 13 is intended to be administered in an amount of 200 mg of the free base, twice daily.

**20.** The compounds according to any one of claims 13 to 18, wherein compound (iii) according to claim 13 is intended to be administered in an amount of 150 mg of the free base, twice daily.

**21.** The compounds according to any one of claims 13 to 18, wherein compound (iii) according to claim 13 is intended to be administered in an amount of 100 mg of the free base, twice daily.

**22.** The compounds according to any one of claims 13 to 18, wherein compound (iv) according to claim 13 is intended to be administered in an amount of 1000 mg of the free base per $m^2$, twice daily at days 1 to 14 of a treatment cycle of 21 days.

**23.** A method of treating metastatic colorectal cancer, the method comprising administering to a patient in need thereof

compound (v): 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof, in combination with compound (vi): 5'-deoxy-5-fluoro-N-[(pentyloxy)carbonyl]-cytidine or a pharmaceutically acceptable salt thereof.

**24.** A method of treating metastatic colorectal cancer, the method comprising administering to a patient in need thereof a pharmaceutical combination consisting essentially of compound (v), and compound (vi),

compound (v): 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof,
compound (vi): 5'-deoxy-5-fluoro-N-[(pentyloxy)carbonyl]-cytidine or a pharmaceutically acceptable salt thereof

**25.** A method of treating metastatic colorectal cancer, the method comprising administering to a patient in need thereof a pharmaceutical combination consisting of compound (v), and compound (vi),

compound (v): 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof,
compound (vi): 5'-deoxy-5-fluoro-N-[(pentyloxy)carbonyl]-cytidine or a pharmaceutically acceptable salt thereof.

**26.** A method of treating metastatic colorectal cancer, the method comprising

- identifying a patient in need of treatment of colorectal cancer
- treating the patient with a combination therapy comprising compound (v), and compound (vi),

compound (v): 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof,
compound (vi): 5'-deoxy-5-fluoro-N-[(pentyloxy)carbonyl]-cytidine or a pharmaceutically acceptable salt thereof.

**27.** The method of any one of the claims 23 to 26, where the patients were refractory or intolerant to standard therapies.

**28.** The method of any one of the claims 23 to 27, where the colorectal cancer is metastatic.

**29.** The method of any one of the claims 23 to 28, where the colorectal cancer is metastatic, where the patients were refractory or intolerant to standard therapies selected from the group consisting of fluoropyrimidine, irinotecan, oxaliplatin, anti-angiogenesis inhibitor and anti-EGFR antibody (if wild-type RAS).

**30.** Use of 3-*Z*-[1-(4-(*N*-((4-methyl-piperazin-1-yl)-methylcarbonyl)-*N*-methyl-amino)-anilino)-1-phenyl-methylene]-6-methoxycarbonyl-2-indolinone or a pharmaceutically acceptable salt thereof for the manufacture of a pharmaceutical composition for the combination therapy of colorectal cancer of a patient, wherein the combination therapy relies in the co-administration of 5'-deoxy-5-fluoro-N-[(pentyloxy)carbonyl]-cytidine or a pharmaceutically acceptable salt thereof,

Europäisches Patentamt
European Patent Office
Office européen des brevets

# EUROPEAN SEARCH REPORT

Application Number
EP 16 17 0498

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br>Y | AWASTHI NIRANJAN ET AL: "Profile of nintedanib in the treatment of solid tumors: the evidence to date", ONCOTARGETS AND THERAPY, vol. 8, 2015, pages 3691-3701, XP002763618,<br>* abstract *<br>* page 3692, right-hand column, line 39 - line 43 *<br>* page 3693, right-hand column, line 16 - line 19 *<br>* page 3699, left-hand column, line 18 - line 20 *<br>----- | 1-30<br><br><br><br><br><br>1-30 | INV.<br>A61K31/496<br>A61P35/00<br>A61P35/04<br>A61K31/513 |
| X<br><br><br><br><br>Y | ROTH GERALD J ET AL: "Nintedanib: From Discovery to the Clinic", JOURNAL OF MEDICINAL CHEMISTRY, vol. 58, no. 3, February 2015 (2015-02), pages 1053-1063, XP002763619,<br>* abstract *<br>* page 1055, left-hand column, line 45 - line 47 *<br>* page 1060, left-hand column, line 42 - line 46 *<br>----- | 30<br><br><br><br><br>1-30 | TECHNICAL FIELDS SEARCHED (IPC)<br>A61K |
| Y | EP 1 824 504 A1 (AVENTIS PHARMA SA [FR]) 29 August 2007 (2007-08-29)<br>* page 3, paragraph 1 - paragraph 3 *<br>* page 5, paragraph 26 *<br>* claim 2 *<br>----- | 1-30 | |

-/--

The present search report has been drawn up for all claims

2

| Place of search | Date of completion of the search | Examiner |
|---|---|---|
| Munich | 31 October 2016 | Terenzi, Carla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 16 17 0498

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VAN CUTSEM E ET AL: "Oral capecitabine vs intravenous 5-fluorouracil and leucovorin: integrated efficacy data and novel analyses from two large, randomised, phase III trials", BRITISH JOURNAL OF CANCER, NATURE PUBLISHING GROUP, GB, vol. 90, no. 6, 22 March 2004 (2004-03-22), pages 1190-1197, XP002340147, ISSN: 0007-0920, DOI: 10.1038/SJ.BJC.6601676 * abstract * ----- | 1-30 | |
| | | | TECHNICAL FIELDS SEARCHED (IPC) |

The present search report has been drawn up for all claims

2

| Place of search | Date of completion of the search | Examiner |
|---|---|---|
| Munich | 31 October 2016 | Terenzi, Carla |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 17 0498

This annex lists the patent family members relating to the patent documents cited in the above-mentioned European search report. The members are as contained in the European Patent Office EDP file on
The European Patent Office is in no way liable for these particulars which are merely given for the purpose of information.

31-10-2016

| Patent document cited in search report | Publication date | Patent family member(s) | Publication date |
|---|---|---|---|
| EP 1824504 A1 | 29-08-2007 | AT 426409 T | 15-04-2009 |
| | | AU 2005311191 A1 | 08-06-2006 |
| | | BR PI0518700 A2 | 02-12-2008 |
| | | CA 2586735 A1 | 08-06-2006 |
| | | CN 101068564 A | 07-11-2007 |
| | | CY 1109181 T1 | 02-07-2014 |
| | | DK 1824504 T3 | 20-07-2009 |
| | | EP 1824504 A1 | 29-08-2007 |
| | | ES 2324233 T3 | 03-08-2009 |
| | | FR 2878749 A1 | 09-06-2006 |
| | | HK 1114769 A1 | 05-08-2011 |
| | | HR P20090336 T1 | 31-07-2009 |
| | | IL 183481 A | 30-09-2013 |
| | | JP 4980236 B2 | 18-07-2012 |
| | | JP 2008521866 A | 26-06-2008 |
| | | KR 20070091130 A | 07-09-2007 |
| | | LU 92202 I2 | 15-11-2013 |
| | | LU 92203 I2 | 15-11-2013 |
| | | PT 1824504 E | 25-06-2009 |
| | | RU 2384344 C2 | 20-03-2010 |
| | | SI 1824504 T1 | 31-08-2009 |
| | | US 2006178305 A1 | 10-08-2006 |
| | | US 2009285841 A1 | 19-11-2009 |
| | | US 2013184205 A1 | 18-07-2013 |
| | | WO 2006059012 A1 | 08-06-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0127081 A
- WO 2004013099 A
- WO 2007141283 A
- WO 2004017948 A
- WO 2004096224 A
- WO 2006067165 A


### Non-patent literature cited in the description


- **TEBUTT et al.** *J.Clin.Oncol.,* 2010, vol. 28, 3191-3198
- A review on capacetabine. *World journal of pharmaceutical research,* vol. 4 (7), 1427-1432
- **SWARBRICK, BOYLANN.** Encyclopedia of pharmaceutical technology. Marcel Dekker, 1990, vol. 2, 269 ff
- **LACHMANN et al.** The Theory and Practice of Industrial Pharmacy. 1976, 404-419
- **JIMERSON R. F. et al.** Soft gelatin capsule update. *Drug Dev. Ind. Pharm.,* 1986, vol. 12 (8-9), 1133-44